# DEMANDE DE BREVET EUROPEEN

(11) **EP 2 441 414 A1**
(43) Date de publication de la demande: **18.04.2012**
(21) Numéro de dépôt: 11184963.4
(22) Date de dépôt: 13.10.2011
(51) Int. Cl.: A61F 2/00, A61B 17/04, A61F 2/08

(54) **Dispositif et système de préparation per-opératoire d'un patch d'implantation à suturer**

(30) Priorité: 11.02.2011 FR 1100431; 13.10.2010 US 392850 P
(71) Demandeur: Tornier, Inc., Edina, MN 55435 (US)
(72) Inventeur: Wilson-Wirth, Corey, San Diego, CA California 92103 (US)
(74) Mandataire: Grand, Guillaume

(57) **Abrégé**

Afin de faciliter la préparation d'un patch d'implantation (2) pour les chirurgiens, en rendant cette préparation plus rapide, plus précise et plus sûre, la présente invention consiste en un dispositif de préparation per-opératoire (1) qui comporte :
- deux éléments (10, 20) qui sont mobiles l'un par rapport à l'autre et qui sont chacun pourvus d'au moins une ouverture traversante (25, 16, 26) de réception d'un fil de suture (4, 6), et
- des moyens mécaniques pour commander le positionnement relatif des deux éléments dans une configuration de service dans laquelle les deux éléments sont sensiblement plaqués l'un contre l'autre de manière à, à la fois, aligner la ou au moins une des ouvertures traversantes de l'un des deux éléments avec la ou au moins une des ouvertures traversantes de l'autre élément et maintenir en place entre eux le patch à préparer.

## Description

La présente invention concerne un dispositif et un système de préparation per-opératoire d'un patch d'implantation à suturer.

Dans le présent document, le terme patch désigne des greffes ou des greffons en pièce, constitués essentiellement de matières biocompatibles, indifféremment biologiques ou synthétiques. De telles pièces sont aujourd'hui de plus en plus utilisées pour reconstituer un tissu originel d'un patient, qui a subi une altération ou un endommagement. Ainsi, ces patchs sont actuellement utilisés, par exemple, en chirurgie orthopédique pour reconstruire une surface osseuse articulaire, ainsi qu'en chirurgie ligamentaire pour réparer des tendons ou des insertions musculaires, telles que l'insertion de la coiffe des rotateurs.

En pratique, pour être efficaces, ces patchs doivent être fixés de manière fiable et pérenne à des tissus anatomiques du patient. Pour ce faire, les chirurgiens utilisent des fils de suture qu'ils passent à travers le patch et qu'ils relient aux tissus du patient. Le cas échéant, ces fils de suture coopèrent avec des ancres solidarisées à un os du patient et/ou sont noués selon divers types de points de suture. Lorsque le patch est de petites dimensions et qu'il n'est associé qu'à un ou deux fils de suture, les chirurgiens peuvent préparer le patch in situ, c'est-à-dire à proximité immédiate des tissus du patient sur lesquels le patch va être suturé, après avoir introduit le patch dans le corps du patient. Toutefois, dès que le patch est un peu plus grand et/ou qu'il va être fixé par davantage de fils de suture, les chirurgiens n'ont pas d'autre choix que de préparer le patch à l'extérieur du corps du patient, typiquement dans un champ stérile délimité sur une table d'intervention dédiée du bloc chirurgical. En effet, on souligne que la préparation des patchs à implanter est réalisée de manière per-opératoire car le chirurgien doit d'abord aborder chirurgicalement le site d'implantation du patch dans le patient pour estimer au mieux l'agencement des fils de suture nécessaires à sa fixation. De plus, lors de la préparation du patch, juste avant son implantation, le chirurgien doit souvent redimensionner ce patch, dans le sens où il doit découper dans une pièce de relativement grandes dimensions un morceau, qui constituera le patch à implanter, dont les dimensions et la forme sont adaptées aux besoins de l'intervention chirurgicale en cours. Il en résulte que, au cours de cette intervention, le chirurgien se voit contraint de manipuler le patch pour, successivement, le redimensionner par découpage, soit à l'aide d'une paire de ciseaux, ce qui rend souvent les bords découpés du patch irréguliers, soit à l'aide d'un scalpel, ce qui nécessite de bien immobiliser le patch lors de l'application du scalpel, puis passer à travers le patch plusieurs fils de suture, ce qui nécessite de tenir et de légèrement tendre le patch, typiquement à l'aide de pinces hémostatiques, pendant que le chirurgien enfonce à travers le patch des aiguilles d'entraînement des fils de suture.

Bien entendu, toutes les manipulations fastidieuses décrites ci-dessus doivent être réalisées de manière aussi aseptique que possible, étant remarqué que, en début d'intervention, le patch est généralement fourni dans un conditionnement stérile.

Le but de la présente invention est de proposer un dispositif qui facilite significativement la préparation d'un patch pour le chirurgien, en rendant cette préparation plus rapide, plus précise et plus sûre.

A cet effet, l'invention a pour objet un dispositif de préparation per-opératoire d'un patch d'implantation à suturer, tel que défini à la revendication 1.

Une des idées à la base de l'invention est de maintenir en place un patch à préparer entre deux éléments superposés, en prenant, en quelque sorte, le patch « en sandwich » mais, bien sûr, sans l'endommager par un excès de contraintes d'immobilisation entre ces deux éléments. Pour ce faire, le dispositif selon l'invention inclut un mécanisme permettant de rapprocher les deux éléments et de les mettre en place de part et d'autre du patch interposé entre eux. Dans certains modes de réalisation de l'invention, ces deux éléments sont avantageusement liés à demeure l'un à l'autre, en étant ainsi facilement manipulables conjointement. Pour que le chirurgien puisse alors faire passer au moins un fil de suture à travers le patch, les deux éléments du dispositif sont troués de part en part de manière que le fil de suture puisse être introduit successivement à travers un des deux éléments, à travers le patch maintenu entre les deux éléments, et à travers le second élément. Bien entendu, dans certains modes de réalisation de l'invention, chacun des deux éléments du dispositif inclut une pluralité d'ouvertures traversantes pour y recevoir un fil de suture, permettant ainsi au chirurgien de choisir l'emplacement du passage du fil de suture, sans avoir à modifier le positionnement relatif entre le patch et les deux éléments. Le dispositif selon l'invention permet ainsi de mettre en place des fils de suture à travers un patch de manière rapide et précise, tout en limitant le risque de blessures lors des manipulations correspondantes du patch, en particulier par des aiguilles d'entraînement des fils de suture, puisque de telles aiguilles sont guidées à travers les ouvertures traversantes des deux éléments du dispositif lorsqu'elles traversent le patch.

Dans certains modes de l'invention, le dispositif permet de mettre en place aussi bien des fils de suture totalement libres, c'est-à-dire des fils de suture qui, avant d'être passés à travers le patch, ne sont liés à rien, ce qui revient à dire que ces fils de sutures sont individuellement indépendants, que des fils de suture déjà liés à un autre objet que le patch, typiquement des fils de suture qui, avant d'être passés à travers le patch, ont déjà été passés à travers ou bien noués à des tissus anatomiques du patient. Cet aspect de l'invention sera mieux compris par la suite.

Des caractéristiques additionnelles avantageuses du dispositif conforme à l'invention, prises isolément ou selon toutes les combinaisons techniquement possibles, sont spécifiées aux revendications dépendantes 2 à 14.

L'invention a également pour objet un système de préparation per-opératoire d'un patch d'implantation à suturer, tel que défini à la revendication 15.

L'invention sera mieux comprise à la lecture de la description qui va suivre, donnée uniquement à titre d'exemple et faite en se référant aux dessins sur lesquels :
- la figure 1 est une vue en perspective d'un premier mode de réalisation d'un dispositif conforme à l'invention, montré dans une configuration initiale de repos ;
- les figures 2 à 4 sont également des vues en perspective du dispositif de la figure 1, les figures 3 et 4 montrant deux étapes successives d'utilisation du dispositif alors que ce dernier est dans une configuration de service, tandis que la figure 2 montre le dispositif dans une configuration intermédiaire entre la configuration initiale de la figure 1 et la configuration de service des figures 3 et 4 ;
- la figure 5 est une coupe dans le plan V de la figure 4 ;
- la figure 6 est une vue analogue à la figure 3, montrant une autre utilisation du dispositif de la figure 1 ; et
- les figures 7 et 8 sont des vues respectivement analogues aux figures 2 et 3, illustrant un second mode de réalisation d'un dispositif conforme à l'invention.

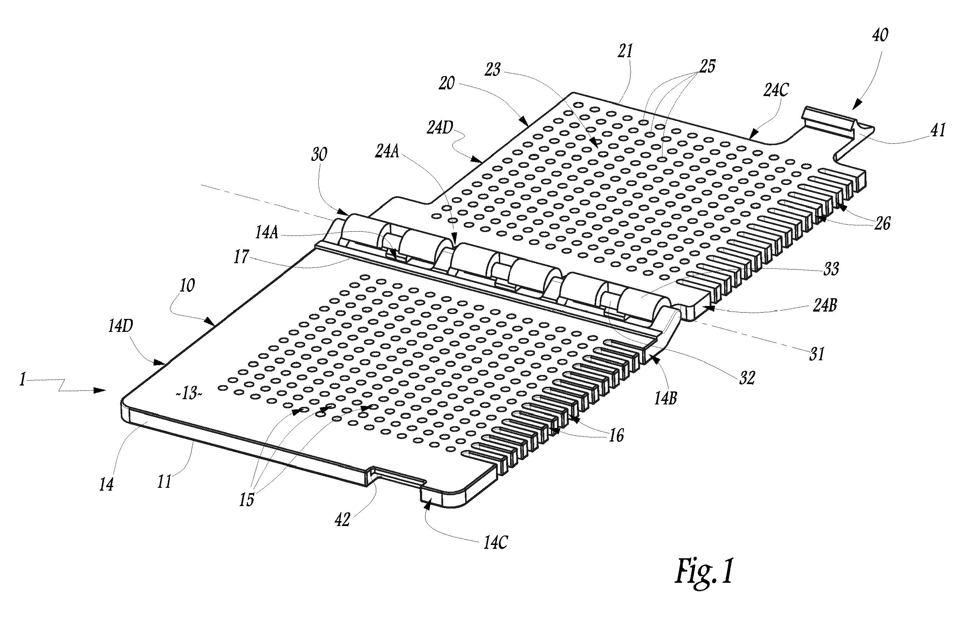

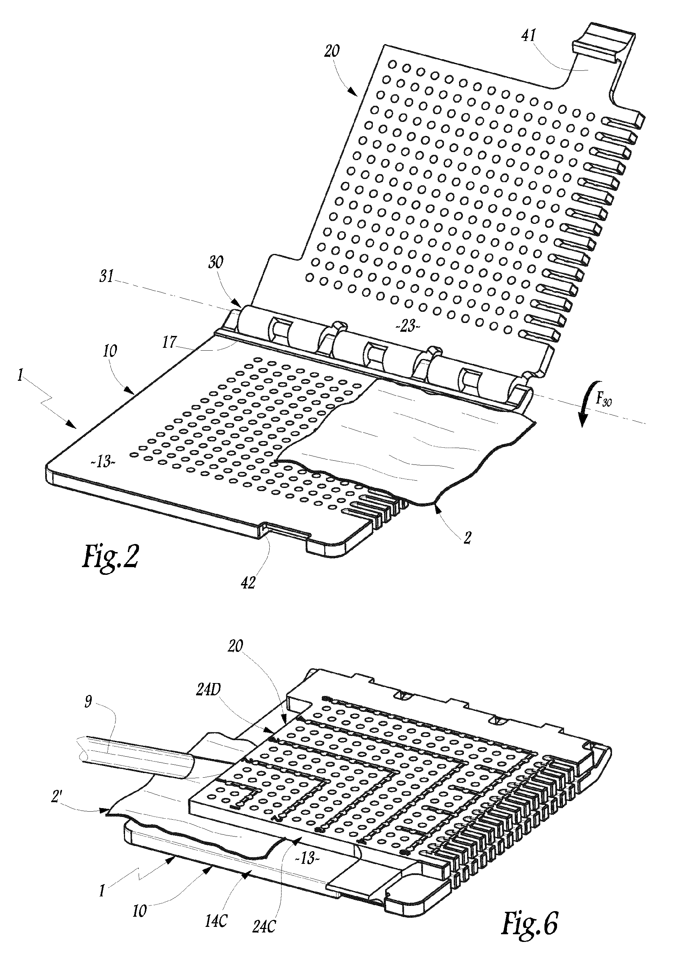

Sur les figures 1 à 6 est représenté un dispositif 1 de préparation d'un patch d'implantation 2, qui est visible, au moins partiellement, sur les figures 2 à 5. Comme expliqué dans la partie introductive du présent document, ce patch 2 comprend, voire, comme ici, consiste en une feuille destinée à être implantée dans les tissus du corps d'un patient au cours d'une intervention chirurgicale, en vue de reconstruire ou de remplacer tout ou partie d'un tissu anatomique originel du patient, tel qu'un tissu osseux, un tissu ligamentaire, etc. A cet effet, le patch 2 est constitué d'une matière biocompatible, le cas échéant non résorbable, et indifféremment biologique ou synthétique. En particulier, la feuille du patch 2 est constituée d'une matrice tissulaire d'origine animale, notamment porcine, ou d'origine humaine, ou bien encore synthétique.

Comme bien visible sur la figure 1, le dispositif 1 comprend essentiellement deux éléments 10 et 20, respectivement en forme de plaque, qui sont assemblés l'un à l'autre par un mécanisme de liaison à charnière 30.

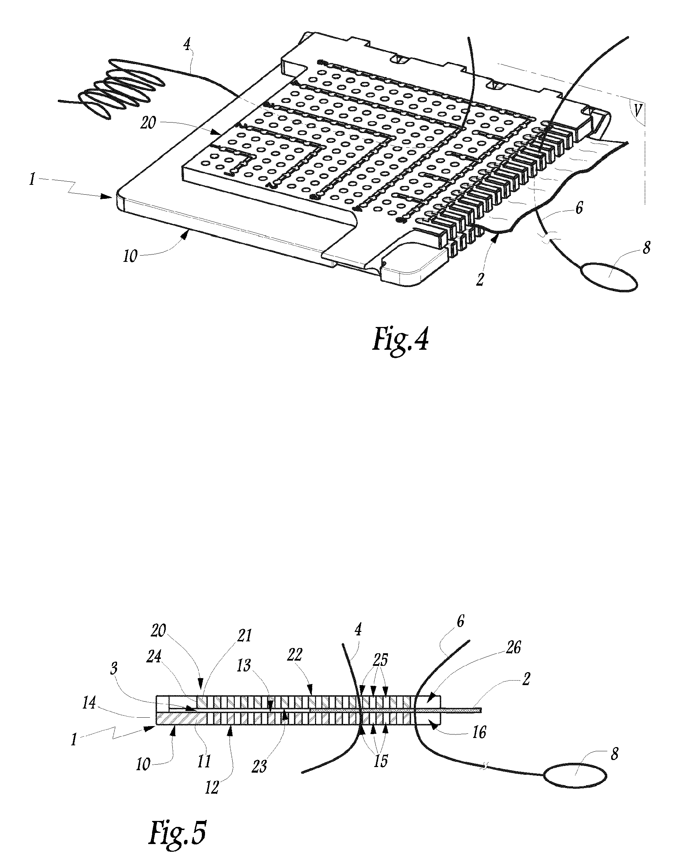

Chaque élément 10, 20 comprend principalement un corps 11, 21, qui se présente globalement sous la forme d'un parallélépipède dont l'épaisseur est significativement plus petite que sa longueur et sa largeur, et qui, dans l'exemple de réalisation considéré ici, est monobloc. Chaque corps 11, 21 définit ainsi deux faces principales planes opposées 12 et 13, 22 et 23 (figure 5), qui sont séparées l'une de l'autre par l'épaisseur du corps, en formant, suivant la périphérie de ce corps, un bord d'extrémité 14, 24. Dans l'exemple de réalisation considéré, chaque bord 14, 24 est constitué de quatre portions globalement rectilignes, qui se succèdent suivant la périphérie du corps 11, 21.

Pour des raisons qui apparaîtront plus loin, chaque corps 11, 21 est avantageusement réalisé en un matériau transparent ou, à tout le moins, translucide.

Le mécanisme 30 relie des portions respectives des bords 14 et 24 des éléments 10 et 20, les portions ainsi reliées étant référencées 14A et 24A sur les figures 1 et 2. Le mécanisme 30 permet, au niveau des portions de bord 14A et 24A agencées de manière sensiblement jointive le long l'une de l'autre, de faire basculer les éléments 10 et 20 l'un vis-à-vis de l'autre, autour d'un axe de charnière 31 qui s'étend de manière parallèle à la direction longitudinale des portions de bord 14A et 24A. En pratique, diverses formes de réalisation sont envisageables pour ce mécanisme 30 : sur les figures, la portion de bord 14A est pourvue d'une tige 32, qui est centrée sur l'axe de charnière 31 et autour de laquelle est montée de manière basculante une fiche 33 solidaire de la portion de bord 24A. Cette forme de réalisation n'est qu'illustrative, dans le sens où une multitude de formes de réalisation sont envisageables pour le mécanisme de liaison à charnière 30.

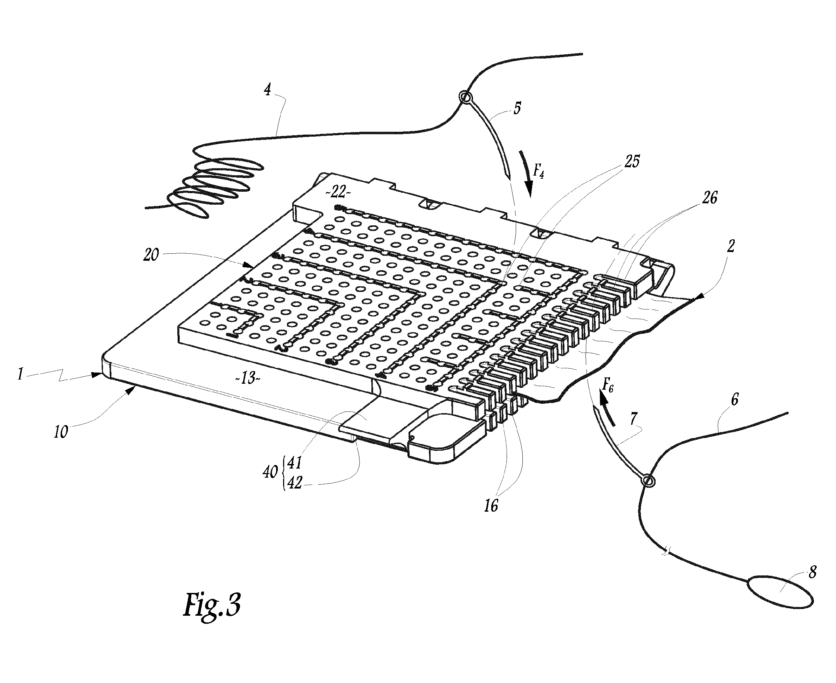

Par actionnement du mécanisme de liaison à charnière 30, les éléments 10 et 20 sont déplaçables l'un vis-à-vis de l'autre entre une configuration de repos, montrée à la figure 1, à une configuration de service, montrée sur les figures 3 à 5, en passant par une configuration intermédiaire montrée à la figure 2. Dans la configuration de repos de la figure 1, les éléments 10 et 20 sont positionnés côte à côte de manière que leurs corps respectifs 11 et 21 s'étendent dans un même plan, leurs faces respectives 12 et 22 étant dirigées vers une même direction, à savoir vers le bas sur la figure 1, tandis que leurs faces respectives 13 et 23 sont tournées toutes les deux en direction opposée, c'est-à-dire vers le haut sur la figure 1. Ainsi, cette configuration du dispositif 1 montrée à la figure 1 peut être qualifiée de configuration ouverte pour le dispositif, ce dernier étant typiquement posé sur un plan de travail horizontal, contre lequel prennent appui les faces 12 et 22 des corps 11 et 21 des éléments 10 et 20.

Dans la configuration de service des figures 3 à 5, les éléments 10 et 20 sont positionnés l'un sur l'autre de manière que leurs corps respectifs 11 et 21 s'étendent dans des plans respectifs distincts et parallèles, en se superposant suivant une direction perpendiculaire aux plans précités. Dans cette configuration de service, qui peut également être qualifiée de configuration fermée, la face 13 du corps 11 de l'élément 10 est agencée à l'aplomb de et est tournée vers la face 23 du corps 21 de l'élément 20, en formant entre ces faces 13 et 23 un espace libre d'interposition 3.

Cet espace libre 3 est dimensionné pour recevoir le patch 2, comme bien visible sur la figure 5. Plus précisément, dans la configuration de service du dispositif 1, le patch 2 est prévu pour être interposé entre les éléments 10 et 20, avec ses deux faces principales opposées respectivement en appui contre la face 13 du corps 11 de l'élément 10 et la face 23 du corps 21 de l'élément 20. Autrement dit, le patch 2 se trouve alors « pris en sandwich » entre les éléments 10 et 20. De cette façon, dans la configuration de service du dispositif 1, les éléments 10 et 20 maintiennent en place entre eux le patch 2, par appui de leurs faces respectives 13 et 23 contre les faces principales opposées de ce patch.

A titre d'option avantageuse, non représenté sur les figures, l'une et/ou l'autre des faces 13 et 23 des éléments 10 et 20 sont texturées ou, plus généralement, conformées en bosses et/ou en creux, de manière à présenter des reliefs contre lesquels le patch 2 est retenu mécaniquement par frottement. De cette façon, l'immobilisation du patch 2 contre les faces 13 et 23 est renforcée.

Comme bien visible sur les figures 1 et 5, chaque élément 10, 20 est pourvu d'une pluralité d'ouvertures traversantes, que l'on peut répartir en un premier groupe d'ouvertures constituées de trous cylindriques 15, 25, et en un second groupe d'ouvertures constituées de fentes 16, 26. Les trous 15, 25 et les fentes 16, 26 ont en commun de traverser de part en part chaque corps 11, 21 en reliant ainsi les faces opposées 12 et 13, 22 et 23 de ce corps. Les trous 15, 25 se distinguent par le fait que leur contour est fermé sur lui-même, c'est-à-dire que, dans un plan de coupe transversale à ces trous, autrement dit dans un plan parallèle aux faces 12 et 13, 22 et 23, la section du trou est entourée, sur toute sa périphérie, par de la matière du corps 11, 21. Les fentes 16, 26 présentent, quant à elles, un contour ouvert, c'est-à-dire que, dans un plan de coupe transversale, leur section n'est que partiellement délimitée, suivant la périphérie de cette section, par de la matière constituant le corps 11, 21, une portion périphérique de cette section étant librement ouverte sur l'extérieur du corps 11, 21, ce qui revient à dire que, en plus de déboucher sur les faces 12 et 13, 22 et 23 du corps 11, 21, les fentes 16, 26 débouchent sur le bord 14, 24 du corps 11, 21. Dans l'exemple de réalisation représenté, les fentes 16, 26 débouchent ainsi sur une portion du bord 14, 24 qui est référencée 14B, 24B et qui est distincte de la portion de bord 14A, 24A le long de laquelle est agencé le mécanisme de liaison à charnière 30.

Lorsque le dispositif 1 est dans sa configuration de service des figures 3 à 5, les trous 15 de l'élément 10 sont alignés avec les trous 25 de l'élément 20, c'est-à-dire que à chacun des trous 15 est associé un des trous 25, en formant ainsi une paire de trous alignés l'un avec l'autre, c'est-à-dire qui s'étendent dans le prolongement l'un de l'autre, en particulier dans le prolongement rectiligne l'un de l'autre, suivant une direction perpendiculaire aux plans dans lesquels s'étendent respectivement les éléments 10 et 20. De la même façon, dans la configuration de service de la figure 3, les fentes 16 sont alignées avec les fentes 26, c'est-à-dire que chacune des fentes 16 est située dans le prolongement d'une des fentes 26, en particulier suivant une direction perpendiculaire aux plans précités.

Dans la forme de réalisation du dispositif 1 montrée sur les figures, les trous 15, 25 de chaque élément 10, 20 sont répartis selon un quadrillage métrique, c'est-à-dire que ces trous sont répartis suivant un motif en lignes et en colonnes, perpendiculaires entre elles, en étant chacun situé, le long d'une de ces lignes et le long d'une de ces colonnes, à une distance préétablie des trous 15, 25 immédiatement voisins, la distance précitée étant avantageusement constante pour toutes les lignes et toutes les colonnes, obtenant ainsi un quadrillage métrique régulier, comme dans l'exemple de réalisation montré sur les figures. Avantageusement, également comme dans l'exemple de réalisation considéré ici, l'espacement entre deux lignes adjacentes et entre deux colonnes adjacentes peut être visuellement quantifié, à l'aide de repères alphanumériques inscrits sur le corps 11, 21 notamment sur la face 12, 22 de ce corps : ainsi, comme représenté sur la figure 3, la face 22 de l'élément 20 porte des repères alphanumériques de ce type, sous la forme de la succession de chiffres « 1, 2, 3, 4, 5 ».

Quant aux fentes 16 et 26, elles sont avantageusement réparties de manière régulière le long des portions de bord 14B et 24B des corps 11 et 21 des éléments 10 et 20. Ainsi, les fentes 16 et les fentes 26 sont réparties en un motif pré-établi s'apparentant à une graduation métrique, la distance entre deux fentes successives le long des portions de bord 14B et 24B étant pré-établie.

L'intérêt des trous 15 et 25, ainsi que des fentes 16 et 26 va être mieux compris en considérant un exemple d'utilisation du dispositif 1, exposé ci-après.

Le contexte général de cet exemple d'utilisation est celui d'une intervention chirurgicale visant à implanter le patch 2 dans un patient, en suturant ce patch à des tissus anatomiques du patient à l'aide de fils de suture.

Initialement, alors que l'intervention chirurgicale précitée est en cours, c'est-à-dire typiquement après que le chirurgien ait abordé le site d'implantation du patch 2, via une incision ou un passage arthroscopique, le chirurgien met en place le dispositif 1 sur un plan de travail, dans sa configuration de repos de la figure 1. En pratique, à ce stade, le dispositif 1 est mis à disposition dans un état aseptique : par exemple, soit le dispositif 1 est un dispositif jetable, qui est livré au chirurgien dans un conditionnement stérile, soit le dispositif 1 est conçu pour être réutilisé et, dans ce cas, avant d'être mis à disposition du chirurgien pour l'intervention en cours, il a préalablement subi un traitement de stérilisation.

Alors que le dispositif 1 est dans sa configuration de repos, le chirurgien se saisit du patch 2, qu'il sort d'un conditionnement stérile, et pose ce patch sur la face 13 du corps 11 de l'élément 10, comme représenté sur la figure 2. Dans la forme de réalisation du dispositif 1 considérée ici, le positionnement du patch 2 sur la face 13 est avantageusement facilité par la présence d'une bordure 17 s'étendant en saillie de la face 13, le long d'au moins une partie de la périphérie de cette face, ici le long de la portion de bord 14A : cette bordure 17 forme une butée de positionnement pour le patch 2, contre laquelle le chirurgien va appliquer la tranche du patch pour s'assurer d'un positionnement relatif prédéterminé entre le patch 2 et la face 13, par exemple pour s'assurer que le patch 2 ne s'étende pas en biais sur cette face 13, mais qu'il s'étend de manière jointive le long de la bordure 17, comme représenté sur la figure 2. On comprendra que la bordure 17 n'est qu'un exemple de réalisation pour la butée de positionnement précitée.

Comme représenté sur la figure 2, le chirurgien rapproche alors l'un de l'autre les éléments 10 et 20, en entraînant en basculement l'élément 20 par rapport à l'élément 10 autour de l'axe de charnière 31, comme indiqué par la flèche F₃₀ sur la figure 2. Le mécanisme 30 guide le mouvement de basculement de l'élément 20 par rapport à l'élément 10, jusqu'à ce que ces éléments atteignent la configuration de service du dispositif 1, comme montré sur les figures 3 à 5.

A titre d'option avantageuse, le mode de réalisation considéré ici pour le dispositif 1 inclut un mécanisme pour bloquer les éléments 10 et 20 l'un vis-à-vis de l'autre dans la configuration de service. En pratique, ce mécanisme de blocage 40 peut prendre des formes de réalisation diverses. Dans l'exemple considéré sur les figures, ce mécanisme 40 inclut une languette 41, qui est solidaire du corps 21 de l'élément 20, en s'étendant en saillie de la portion de bord 24C opposée à la portion de bord 24A, et qui est conçue pour coopérer par déformation souple relative et par complémentarité de formes avec une encoche dédiée 42 délimitée dans le corps 11 de l'élément 10, le long de sa portion de bord 14C opposée à la portion de bord 14A. Autrement dit, la languette 41 est adaptée pour venir se clipser dans l'encoche 42 lorsque le dispositif 1 est dans sa configuration de service, immobilisant ainsi les éléments 10 et 20 l'un vis-à-vis de l'autre, sauf à appliquer une action spécifique de déverrouillage sur la languette 41, pour la désengager de l'encoche 42 et ainsi autoriser le basculement de l'élément 20, en sens inverse de celui montré à la figure 2.

Lorsque le dispositif 1 passe ainsi dans sa configuration de service, comme montré sur la figure 3, le patch 2 se trouve interposé entre les éléments 10 et 20, avec la face 13 de l'élément 10 et la face 23 de l'élément 20 s'appuyant respectivement sur ses faces principales opposées. Autrement dit, le plaquage des corps 11 et 21 l'un contre l'autre permet de maintenir en place entre ces corps le patch 2, comme expliqué plus haut.

Le chirurgien se saisit alors d'un premier fil de suture 4 qu'il va faire passer à travers le patch 2, en l'introduisant successivement dans l'un des trous 25 de l'élément 20 et le trou 15 de l'élément 10, situé dans l'alignement du trou 25 précité. Bien entendu, on comprend que le diamètre du fil 4 est prévu inférieur à celui des trous 15 et 25 pour permettre le passage du fil à travers eux. En pratique, dans la mesure où le fil 4 doit traverser de part en part le patch 2 pour passer du trou 25 au trou 15, le chirurgien utilise également une aiguille 5, à laquelle le fil 4 est relié et qui sert ainsi à entraîner ce fil à travers successivement le trou 25, le patch 2 et le trou 15, comme indiqué par la flèche F₄ sur la figure 3. Les figures 4 et 5 illustrent la situation après cette mise en place du fil de suture 4.

Dans l'exemple d'utilisation considéré ici, le chirurgien utilise également un second fil de suture 6, distinct du fil 4. Comme représenté sur les figures 3 à 5, le fil 6 s'utilise sensiblement de la même façon que le fil 4, notamment à l'aide d'une aiguille d'entraînement 7, à la différence notable que, pour des raisons développées ci-après, le fil 6 est prévu pour être passé successivement à travers l'une des fentes 16, le patch 2 et la fente 26 située dans l'alignement de la fente 16 précitée. La mise en place du fil 6 est indiquée schématiquement par la flèche F₆ sur la figure 3.

La manipulation des aiguilles 5 et 7, pour les faire passer à travers les éléments 10 et 20, est facilitée par le fait que les corps 11 et 21 de ces éléments sont transparents ou translucides.

A ce stade, avant d'expliquer la différence entre les fils de suture 4 et 6, on remarquera que le sens de passage de chaque fil de suture à travers le patch 2 n'a aucune importance : ainsi, à l'opposé de ce qui a été décrit précédemment, il est tout à fait envisageable de faire passer le fil 4 successivement à travers l'un des trous 15 de l'élément 10, puis le patch 2, puis le trou 25 de l'élément 20 situé dans l'alignement du trou 15 précité. De la même façon, il est envisageable de faire passer le fil de suture 6 à travers successivement l'une des fentes 26 de l'élément 20, le patch 2, puis la fente 16 située dans l'alignement de la fente 26 précitée.

Les fils de suture 4 et 6 se distinguent l'un de l'autre par le fait que, d'un côté, le fil 4 est un fil totalement libre, dans le sens où, avant d'être passé à travers le patch 2, ce fil est totalement indépendant de tout autre objet : autrement dit, le fil de suture 4 se résume exclusivement à un segment d'élément filaire. A l'inverse, le fil de suture 6 est, à l'une de ses extrémités, déjà relié à un objet 8 avant d'être passé à travers le patch 2, comme décrit plus haut. En pratique, l'objet précité 8 peut être un tissu anatomique du patient ou une ancre de suture : dans tous les cas, cet objet 8 présente une dimension transversale largement supérieure au diamètre du fil 6, ce qui explique pourquoi ce fil 6 doit être passé par une paire de fentes 16 et 26, et non par une paire de trous 15 et 25, pour des raisons développées un peu plus loin.

Avant de poursuivre la description de l'exemple d'utilisation, on remarquera que, eu égard à la pluralité des trous 15 et 25 et à la pluralité des fentes 16 et 26, le chirurgien a un choix important quant à l'emplacement de passage des fils 4 et 6 à travers le patch 2. D'ailleurs, en ce qui concerne le fil 4, le chirurgien met avantageusement à profit la répartition selon un motif quadrillé des trous 15 et 25 pour choisir avec précision le positionnement du passage du fil 4 à travers le patch 2, notamment en tenant compte de l'utilisation qu'il compte avoir du fil 4 lors de l'implantation du patch 2 dans le patient. En effet, on gardera à l'esprit que les manipulations des fils de suture 4 et 6 sont réalisées par le chirurgien alors que celui-ci a déjà abordé le site d'implantation du patch 2 et qu'il a donc estimé des emplacements appropriés pour faire passer ces fils 4 et 6 à travers du patch 2, en vue de l'implantation de ce dernier.

Par ailleurs, dans la mesure où plusieurs fentes 16 et plusieurs fentes 26 sont réparties régulièrement le long des portions de bord 14B et 24B des corps 11 et 21 des éléments 10 et 20, le fil de suture 6, qui est passé à travers le patch 2 grâce à ces fentes 16 et 26, peut être doublé, moyennant l'utilisation de deux paires de fentes alignées 16 et 26 : autrement dit, ces fentes 16 et 26 permettent de faire passer à travers le patch 2 un double fil présentant un point de suture de matelassier.

L'exemple d'utilisation du dispositif 1 prend fin, de manière que le chirurgien puisse récupérer le patch 2 avec les fils de suture 4 et 6 passés à travers lui.

Pour ce faire, le chirurgien déverrouille le mécanisme 40 et bascule l'élément 20 par rapport à l'élément 10 autour de l'axe de charnière 31, de manière à passer le dispositif 1 de sa configuration de service à sa configuration de repos de la figure 1, en passant par la configuration intermédiaire de la figure 2. Le chirurgien peut alors se saisir du patch 2 et l'éloigner du dispositif 1, tout en maintenant à travers le patch 2 les fils de suture 4 et 6 : pour ce qui concerne le fil de suture 4, il suffit de faire coulisser le fil à travers, d'un côté du patch, le trou 15 à travers lequel le fil a été précédemment passé et, du côté opposé du patch, dans le trou 25 à travers lequel le fil a été précédemment passé, jusqu'à ce que les extrémités libres de ce fil passent ainsi à travers les trous précités 15 et 25, désengageant ainsi totalement le fil 4 des éléments 10 et 20. En ce qui concerne le fil de suture 6, le fait que les fentes 16 et 26, à travers lesquelles ce fil 6 est passé, présente un contour ouvert est mis à profit pour dégager le fil 6 des éléments 10 et 20, en passant ce fil par l'ouverture des fentes 16 et 26. On comprend ainsi pourquoi, dans la mesure où le fil de suture 6 est lié à l'objet 8, il n'aurait pas été possible de le dégager du dispositif 1 si ce fil avait été passé à travers les trous 15 et 25. Bien entendu, à l'inverse, un fil de suture similaire au fil 4 peut être passé à travers des fentes 16 et 26, le dégagement de ce fil de suture vis-à-vis des éléments 10 et 20 pouvant être réalisé indifféremment à la façon de ce qui a été décrit pour le fil 4 ou à la façon de ce qui vient d'être décrit pour le fil 6.

La figure 6 illustre une possibilité optionnelle d'utilisation du dispositif 1, permettant de facilement redimensionner un patch 2' similaire au patch 2 décrit jusqu'ici. Pour ce faire, comme représenté sur la figure 6, le patch 2' est mis en place dans le dispositif 1 sensiblement de la même façon que le patch 2, à la différence que le patch 2' est positionné entre les éléments 10 et 20 de manière à déborder non pas des portions de bords 14B et 24B comme le patch 2, mais pour déborder des portions de bord 14C et 24C, ainsi que des portions de bord 14D et 24D, opposées aux portions de bord 14B et 24B. Ce positionnement du patch 2' présente un intérêt car, dans le mode de réalisation considéré ici, les portions de bord 24C et 24D de l'élément 20 ne s'étendent pas en affleurement des portions de bords 14C et 14D de l'élément 10 lorsque le dispositif 1 dans sa configuration de service, contrairement aux portions de bord 14B et 24B sur lesquels débouchent respectivement les fentes 16 et 26. Autrement dit, lorsque le dispositif 1 est dans sa configuration de service, la face 13 du corps 11 de l'élément 10 n'est pas recouverte en totalité par la face 23 du corps 21 de l'élément 20, mais, au contraire, une partie de cette face 13 reste découverte, comme bien visible sur la figure 6. Il en résulte que les portions de bords 24C et 24D du corps 21 de l'élément 20 sont disposées à l'aplomb de la face 13 de l'élément 10, avec interposition entre elles du patch 2'. Moyennant une conformation adéquate, ces portions de bord 24C et 24D peuvent alors être utilisées pour guider l'application d'un outil de coupe 9, tel qu'un scalpel, permettant ainsi de redimensionner de manière per-opératoire le patch 2'.

Avantageusement, ce redimensionnement du patch 2' peut être facilement quantifié par le chirurgien, en s'aidant du motif quadrillé que forment les trous 25 de l'élément 20 : ainsi, dans l'exemple montré à la figure 6, l'application de l'outil de coupe 9 le long des portions de bords 24C et 24D permet de découper, dans le patch 2' initial, un morceau de patch rectangulaire, présentant une longueur de trois unités et une largeur de deux unités.

Une fois que ce morceau de patch 2' est découpé, il peut être préparé de la même façon que le patch 2 en regard des figures 1 à 5, le cas échéant en modifiant sa position vis-à-vis des éléments 10 et 20 après les avoir repassés dans leur configuration de repos.

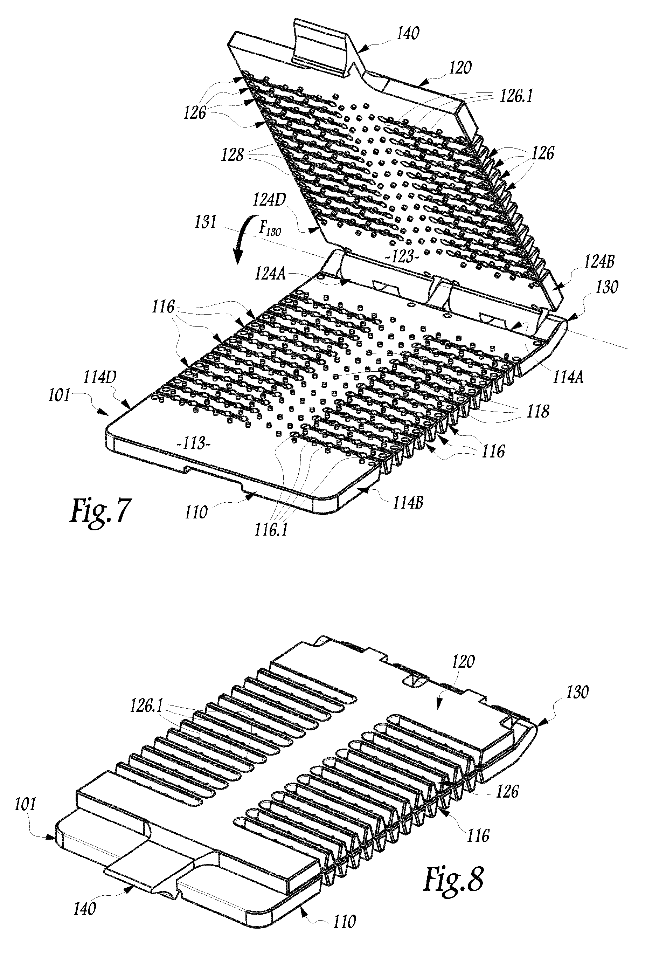

Sur les figures 7 et 8 est représentée une variante de réalisation du dispositif 1, référencée 101. Ce dispositif 101 a la même finalité que le dispositif 1, à savoir préparer un patch d'implantation similaire aux patchs 2 et 2' décrits jusqu'ici. Ceci étant dit, pour des raisons de visibilité et de simplification, le dispositif 101 est montré sur les figures 7 et 8 sans être associé à un tel patch d'implantation.

De manière similaire au dispositif 1, le dispositif 101 comprend essentiellement des éléments 110 et 120, respectivement en forme de plaque, qui sont assemblés l'un à l'autre par un mécanisme de liaison à charnière 130. Par actionnement de ce mécanisme 130, les éléments 110 et 120 sont déplaçables l'un vis-à-vis de l'autre entre une configuration de repos et une configuration de service, respectivement similaires à la configuration de repos et à la configuration de service décrites plus haut pour le dispositif 1. Ainsi, la configuration de service associée au dispositif 110 est montrée à la figure 8, tandis que la figure 7 montre une configuration intermédiaire entre les configurations de repos et de service du dispositif 101. Comme décrit précédemment, pour le mode de réalisation considéré sur les figures 7 et 8, le basculement de l'élément 120 par rapport à l'élément 110 autour de l'axe de charnière 131, qui est défini par le mécanisme 130 et qui s'étend parallèlement à des portions 114A et 124A des bords périphériques respectifs 114 et 124 des éléments 110 et 120, est indiqué par la flèche F₁₃₀ sur la figure 7.

Avant de s'intéresser plus en détail aux différences entre le dispositif 1 et le dispositif 101, on notera que le dispositif 101 inclut un mécanisme de blocage 140 qui, dans l'exemple de réalisation considéré sur les figures 7 et 8, est fonctionnellement et structurellement similaire au mécanisme de blocage 40 du dispositif 1.

Une première différence significative entre les dispositifs 1 et 101 a trait à la nature des ouvertures traversantes dont est pourvu chacun des éléments 110 et 120. En effet, comme bien visible sur la figure 7, chacun des éléments 110 et 120 est dépourvu de trous cylindriques, similaires aux trous 15 et 25 du dispositif 1, mais est uniquement pourvu de fentes 116, 126, qui sont fonctionnellement similaires respectivement aux fentes 16 et 26 du dispositif 1.

Ceci étant dit, à la différence des fentes 16, 26 débouchant toutes sur la même portion 14B, 24B du bord périphérique 14, 24 de l'élément 10, 20 du dispositif 1, les fentes 116, 126 peuvent être réparties en deux groupes distincts, à savoir un premier groupe de fentes, débouchant toutes sur une même portion 114B, 124B du bord périphérique 114, 124 de l'élément 110, 120, et un second groupe de fentes débouchant toutes sur une même portion 114D, 124D du bord 114, 124, qui est opposée à la portion de bord précité 114B, 124B.

Pour chacun des deux groupes de fentes 116 et 126, définis ci-dessus pour chacun des deux éléments 110 et 120, les fentes sont avantageusement réparties de manière régulière le long des portions de bord correspondantes 114B, 114D, 124B et 124D des corps des éléments 110 et 120. Ainsi, chacun des groupes précités de fentes 116 et 126 fourni une graduation métrique le long des portions de bord correspondantes, la distance entre deux fentes successives le long de ces portions de bord étant pré-établie.

Une seconde différence significative entre les dispositifs 1 et 101 est liée à la dimension longitudinale des fentes 116 et 126, comparée à celle des fentes 16 et 26 du dispositif 1. Plus précisément, comme bien visible sur les figures 7 et 8, chacune des fentes 116 et 126 s'étend, depuis la portion de bord correspondante 114B, 114D, 124B ou 124D, sur une partie substantielle du corps de l'élément correspondant 110 ou 120. Ainsi, à titre d'exemple non limitatif, chacune des fentes 116 et 126 s'étend sur plus du tiers, voire presque la moitié du corps de l'élément correspondant 110 ou 120, suivant la direction reliant les portions de bord 114B et 114D ou reliant les portions de bord 124B et 124D.

A titre d'option particulièrement avantageuse, en lien avec la grande étendue en longueur des fentes 116 et 126, chacune d'elles est pourvue de graduations 116.1, 126.1 réparties de manière régulière suivant la direction longitudinale de la fente. Dans l'exemple de réalisation considéré sur les figures 7 et 8, ces graduations 116.1, 126.1 consistent en une série de cinq reliefs en creux, délimités dans les bords opposés de la fente correspondante 116, 126. Dans la mesure où la distance entre deux de ces graduations, qui se suivent le long de la fente correspondante 116, 126, est pré-établie, on comprend que le chirurgien dispose d'une indication visuelle per-opératoire pour repérer dimensionnellement un patch à préparer avec le dispositif 101, le long de la fente correspondante 116, 126.

Par ailleurs, comme déjà évoqué à titre d'option avantageuse non représentée pour le dispositif 1, les faces 113 et 123 des éléments 110 et 120, entre lesquelles un patch à préparer par le dispositif 101 se trouve « pris en sandwich » dans la configuration de service du dispositif, sont pourvues de picots saillants, respectivement référencés 118 et 128. Comme bien visible sur la figure 7, ces picots 118 et 128 sont répartis sur sensiblement toutes les faces 113 et 123, notamment entre les deux groupes précités de fentes 116 ou 126 évoqués plus haut, ainsi qu'entre les fentes 116 et 126 de chacun ce ces groupes. De cette façon, dans la configuration de service du dispositif 101, un patch maintenu en place entre les éléments 110 et 120 est fermement retenu par frottement contre les faces 113 et 123.

L'utilisation du dispositif 101 est similaire à celle du dispositif 1. En particulier, dans la configuration de service montrée à la figure 8, chacune des fentes 116 est alignée avec l'une des fentes 126 de sorte qu'un fil de suture peut être entraîné, à l'aide d'une aiguille, dans au moins une paire d'une des fentes 116 et d'une des fentes 126, alignées l'une avec l'autre. En tenant compte des explications fournies en lien avec le dispositif 1, on comprend que le fil de suture passé à travers successivement l'un et l'autre des éléments 110 et 120, via une des fentes 116 et une des fentes 126, alignées l'une avec l'autre, peut aussi bien être un fil de suture similaire au fil 6 décrit plus haut, qu'un fil de suture similaire au fil 4 décrit plus haut. Dans tous les cas, les graduations 116.1 et 126.1 aident le chirurgien à repérer dans l'espace, vis-à-vis du patch maintenu entre les éléments 110 et 120, l'emplacement de passage du fil de suture à travers ce patch.

Divers aménagements et variantes aux dispositifs 1 et 101 décrits jusqu'ici sont par ailleurs envisageables. A titre d'exemples :
- les éléments 10 et 20, ainsi que les éléments 110 et 120, peuvent présenter d'autres formes que des plaques, du moment qu'ils permettent d'être rapprochés l'un de l'autre et de maintenir entre eux un patch à préparer ; et, même pour les formes de réalisation dans lesquelles ces éléments s'apparentent à des plaques, ces dernières ne présentent pas nécessairement un contour extérieur rectangulaire comme dans les exemples illustrés sur les figures, d'autres formes de contour pouvant être envisagés, telles que des formes circulaires, semi-circulaires, etc. ;
- la commande du positionnement relatif entre les éléments 10 et 20, ainsi qu'entre les éléments 110 et 120, peut être assurée par d'autres moyens mécaniques que le mécanisme à charnière 30 ou 130, l'important est que ces moyens mécaniques permettent de modifier la position relative de ces éléments, pour les passer ainsi d'une configuration ouverte, dans laquelle un patch à préparer peut être facilement déposé sur l'un de ces éléments, sans être gêné par l'autre, à une position de service, dans laquelle les deux éléments sont suffisamment rapprochés l'un de l'autre pour maintenir en place entre eux le patch ;
- plutôt que d'être planes, les faces 13 et 23 des éléments 10 et 20, ainsi que les faces 113 et 123 des éléments 110 et 120, qui s'appuient respectivement sur les deux faces principales opposées du patch à préparer, peuvent présenter d'autres géométries, du moment que ces faces soient complémentaires de manière à maintenir en place entre elles le patch ;
- d'autres formes d'ouvertures traversantes que les trous cylindriques 15 et 25 et que les fentes 16 et 26, ainsi que les fentes 116 et 126, peuvent être envisagées ; en particulier, on peut citer le cas de trous présentant un contour, fermé ou ouvert, multilobé, notamment un contour ayant la forme du symbole « + » : de tels trous à contour multilobé permettent de faire passer à travers le patch 2 des fils de suture à brins multiples, liés entre eux par des points de suture spécifiques, tel que le point de suture de Mason-Allen ; et/ou
- bien entendu, le nombre d'ouvertures traversantes prévues dans chacun des éléments 10 et 20, ainsi que dans chacun des éléments 110 et 120, peut être modifié ; en particulier, on peut envisager le cas où chacun des éléments 10 et 20 ou chacun des éléments 110 et 120 n'est pourvu que d'une seule ouverture traversante, par exemple que d'un seul trou cylindrique ou bien que d'une seule fente débouchante.

## Revendications

1. Dispositif (1 ; 101) de préparation per-opératoire d'un patch d'implantation à suturer (2 ; 2'), comportant :
- deux éléments (10, 20 ; 110, 120) qui sont mobiles l'un par rapport à l'autre et qui sont chacun pourvus d'au moins une ouverture traversante (15, 25, 16, 26 ; 116, 126) de réception d'un fil de suture (4, 6), et
- des moyens mécaniques (30 ; 130) pour commander le positionnement relatif des deux éléments (10, 20 ; 110, 120) dans une configuration de service dans laquelle les deux éléments sont sensiblement plaqués l'un contre l'autre de manière à, à la fois, aligner la ou au moins une des ouvertures traversantes (15, 16 ; 116) de l'un (10 ; 110) des deux éléments avec la ou au moins une des ouvertures traversantes (25, 26 ; 126) de l'autre élément (20 ; 120) et maintenir en place entre eux un patch à préparer (2 ; 2').

2. Dispositif suivant la revendication 1, **caractérisé en ce qu'**au moins l'un des deux éléments (10, 20 ; 110, 120) est réalisé en un matériau transparent ou translucide.

3. Dispositif suivant l'une des revendications 1 ou 2, **caractérisé en ce que** la ou au moins l'une des ouvertures traversantes (15, 25) de chacun des deux éléments (10, 20) présente un contour fermé sur lui-même.

4. Dispositif suivant l'une quelconque des revendications précédentes, **caractérisé en ce que** la ou au moins l'une des ouvertures traversantes (16, 26 ; 116, 126) de chacun des deux éléments (10, 20 ; 110, 120) présente un contour ouvert, débouchant sur un bord péripéhrique (14, 24 ; 114, 124) de l'élément.

5. Dispositif suivant l'une des revendications 1 ou 2, **caractérisé par** plusieurs ouvertures traversantes (116, 126) pour chacun des deux éléments (110, 120), lesquelles ouvertures traversantes présentent toutes un contour ouvert, débouchant sur un bord périphérique (114, 124) de l'élément correspondant.

6. Dispositif suivant l'une des revendications 4 ou 5, **caractérisé en ce que** ledit contour ouvert présente une forme allongée, à l'une des extrémités longitudinales de laquelle ce contour débouche sur ledit bord périphérique (14, 24 ; 114, 124) de l'élément correspondant (10, 20 ; 110, 120), de sorte que la ou chaque ouverture traversante correspondante (16, 26 ; 116, 126) consiste essentiellement en une fente.

7. Dispositif suivant la revendication 6, **caractérisé en ce que** la ou chaque ouverture traversante (116, 126) est pourvue, suivant la direction longitudinale de son contour ouvert, d'un motif pré-établi (116.1, 126.1) d'aide per-opératoire au repérage dimensionnel du patch à préparer, tel que des graduations réparties le long de l'ouverture traversante.

8. Dispositif suivant l'une quelconque des revendications précédentes, **caractérisé en ce qu'**au moins l'un des deux éléments (10, 20 ; 110, 120) est pourvu de plusieurs ouvertures traversantes (15, 16, 25, 26 ; 116, 126), qui sont réparties selon un motif pré-établi, tel qu'un quadrillage métrique ou une graduation métrique, d'aide per-opératoire au repérage dimensionnel du patch à préparer (2 ; 2').

9. Dispositif suivant l'une quelconque des revendications précédentes, **caractérisé en ce que** les deux éléments (10, 20 ; 110, 120) présentent respectivement des faces d'appui (13, 23 ; 113, 123), qui sont complémentaires l'une de l'autre, notamment des faces sensiblement planes, sur lesquelles débouchent la ou les ouvertures respectives (15, 16, 25, 26 ; 116, 126) de ces éléments, et qui sont appliquées l'une sur l'autre, avec interposition du patch à préparer (2 ; 2'), lorsque les éléments sont dans la configuration de service.

10. Dispositif suivant la revendication 9, **caractérisé en ce qu'**au moins l'une (13) des faces d'appui (13, 23) est pourvue d'une butée (17) de positionnement du patch (2 ; 2') sur cette face d'appui.

11. Dispositif suivant l'une des revendications 9 ou 10, **caractérisé en ce qu'**au moins l'une des faces d'appui (13, 23 ; 113, 123) est pourvue d'au moins un relief (118, 128) de retenue du patch (2 ; 2') par frottement.

12. Dispositif suivant la revendication 11, **caractérisé par** plusieurs reliefs de retenue (118, 128) pour chacun des deux éléments (110, 120), ces reliefs de retenue consistant essentiellement en des picots en saillie du reste de la face d'appui correspondante (113, 123) et répartis sur sensiblement toute cette face d'appui.

13. Dispositif suivant l'une quelconque des revendications 9 à 12, **caractérisé en ce que** chacune des faces d'appui (13, 23) est délimitée par un bord périphérique (14, 24) de l'élément (10, 20), et **en ce que**, dans la configuration de service, une partie de la face d'appui (13) d'un premier (10) des deux éléments (10, 20) n'est pas couverte par la face d'appui (23) du second élément (20) de sorte que le bord périphérique (24) de ce second élément inclut au moins une portion (24C, 24D) qui est disposée à l'aplomb de la face d'appui (13) du premier élément (10), cette portion étant au moins en partie conformée pour guider l'application per-opératoire d'un outil de redimensionnement du patch à préparer (2'), tel qu'un outil de coupe (9).

14. Dispositif suivant l'une quelconque des revendications précédentes, **caractérisé en ce que** le dispositif (1 ; 101) comporte un mécanisme (40 ; 140) de blocage des éléments (10, 20 ; 110, 120) l'un vis-à-vis de l'autre dans la configuration de service.

15. Système de préparation per-opératoire d'un patch d'implantation à suturer (2 ; 2'), comportant :
- un dispositif (1 ; 101) conforme à l'une quelconque des revendications précédentes,
- au moins un fil de suture (4, 6) à même d'être introduit dans les ouvertures traversantes (15, 16, 25, 26 ; 116, 126) des deux éléments (10, 20 ; 110, 120) du dispositif (1 ; 101), et
- au moins une aiguille (5, 7) d'entraînement du fil de suture (4, 6) dans les ouvertures traversantes des deux éléments du dispositif.
